# FASCICULE DE BREVET EUROPEEN

(11) **EP 1 268 609 B1**
(45) Date de publication et mention de la délivrance du brevet: **17.05.2006**
(21) Numéro de dépôt: 01907819.5
(22) Date de dépôt: 15.02.2001
(51) Int. Cl.: C08G 73/02, A61K 48/00, C12N 15/87

(54) **PROCEDE DE PREPARATION DE POLYALKYLENIMINES FONCTIONNALISES, COMPOSITIONS LES CONTENANT ET LEUR UTILISATIONS**
VERFAHREN ZUR HERSTELLUNG VON FUNKTIONALIERTEN POLYALKYLENIMINEN, DIESE ENTHALTENDE ZUSAMMENSTELLUNGEN UND IHRE VERWENDUNGEN
METHOD FOR PREPARING FUNCTIONALISED POLYALKYLENIMINES, COMPOSITION CONTAINING SAME AND USES THEREOF

(30) Priorité: 18.02.2000 FR 0002059; 12.05.2000 US 203907 P
(43) Date de publication de la demande: 02.01.2003
(73) Titulaire: CENTELION, 94400 Vitry Sur Seine (FR)
(72) Inventeur: LECLERC, Françoise, F-91440 Bures sur Yvette (FR); HERSCOVICI, Jean, F-75013 Paris (FR); SCHERMAN, Daniel, F-75012 Paris (FR)
(74) Mandataire: Schmitz, Jean-Marie
(86) Numéro de dépôt international: PCT/FR2001/000460
(87) Numéro de publication internationale: WO 2001/060890

(56) Documents cités:
- EP-A- 0 905 254
- WO-A-97/42285
- DE-A- 19 726 186
- US-A- 5 962 400
- LEQUERCQ ET AL.: "Synthesis of glycosylated Polyethylenimine with reduced toxicity and high transfecting efficiency" BIOORGANIC & MEDICINAL CHEMISTRY LETTERS, vol. 10, 29 mars 2000 (2000-03-29), pages 1233-1235, XP000952548
- ZANT M -A ET AL: "IN VITRO GENE DELIVERY TO HEPATOCYTES WITH GALACTOSYLATED POLYETHYLENIMINE" BIOCONJUGATE CHEMISTRY,US,AMERICAN CHEMICAL SOCIETY, WASHINGTON, vol. 8, no. 6, 1 novembre 1997 (1997-11-01), pages 839-844, XP000725213 ISSN: 1043-1802 cité dans la demande

## Description

La présente invention se rapporte à un procédé de préparation de polyalkylènimines fonctionnalisés qui sont utiles pour la formulation d'acides nucléiques destinés à être transfectés dans des cellules.

Avec le développement des biotechnologies, la possibilité de transférer efficacement des acides nucléiques dans les cellules est devenue une technique de base avec de nombreuses applications biotechnologiques. Il peut s'agir du transfert d'acides nucléiques dans des *cellules in vitro,* par exemple pour la production de protéines recombinantes, ou au laboratoire pour l'étude de la régulation de l'expression des gènes, le clonage de gènes ou tout autre manipulation impliquant l'ADN. Il peut également s'agir du transfert d'acides nucléiques dans des cellules *in vivo,* par exemple pour la réalisation de vaccins, des études de marquage ou également des approches thérapeutiques. Il peut encore s'agir du transfert de gènes dans des cellules prélevées d'un organisme, en vue de leur réadministration ultérieure, par exemple pour la création d'animaux transgéniques.

Actuellement, le moyen le plus répandu pour transférer des gènes dans des cellules est l'utilisation de vecteurs viraux. Mais ceux-ci n'étant pas complètement dénués de risques, plusieurs autres méthodes basées sur l'emploi de vecteurs synthétiques ont été proposées. Ces vecteurs synthétiques ont deux fonctions principales : complexer et compacter l'acide nucléique à transfecter, et promouvoir son passage à travers la membrane plasmique et éventuellement à travers les deux membranes nucléaires.

Plusieurs familles de vecteurs synthétiques ont ainsi été proposées. Parmi celles-ci, les polymères cationiques tels que les polyalkylènimines sont particulièrement intéressants. Ils se sont montrés en effet relativement efficaces lors de la transfection d'acides nucléiques, notamment *in vivo.,* et qu'ils présentent en outre une toxicité relativement faible. Il a également été observé que les complexes qu'ils forment avec les acides nucléiques (également appelés « polyplexes ») diffusent relativement bien hors du site d'injection (J.S. Remy et al. ; *Advanced Drug Delivery Reviews,* 30, 1998, pp. 85-95).

Par ailleurs, il semble à ce jour essentiel de pouvoir disposer de vecteurs capables de cibler un acide nucléique approprié vers un organe, un tissu, un type cellulaire ou encore un compartiment cellulaire spécifique, car il est important de pouvoir s'assurer que l'acide nucléique transfecté interagit efficacement avec les cellules cibles sans diffuser défavorablement dans le reste de l'organisme. Le but visé est d'éviter toute action non-spécifique des acides nucléiques sur des cellules autres que les cellules cibles. Il a ainsi été montré que le polyéthylènimine galactosylé est un vecteur efficace pour le transfert *in vitro* de plasmides dans les cellules portant le récepteur cellulaire (lectine) correspondant au galactose (Zanta et al., *Bioconj. Chem.,* 8(2), 1997, p. 839 ; T. Bettinger et al., *Bioconj. Chem., 1999).*

Jusqu'à présent, les polymères tels que par exemple le polyéthylènimine galactosylé, étaient obtenus par action d'un oligosaccharide avec le polyéthylènimine en présence de cyanoborohydrure de sodium. Cependant, ce réactif présente l'inconvénient d'être coûteux et surtout très toxique. Le risque d'une présence potentielle d'ions cyanure résiduels dans le produit final qui est relativement cationique interdit toute possibilité d'utilisation pharmaceutique. En outre, la recherche d'un procédé de préparation alternatif est restée jusque-là infructueuse car les polyalkylènimines sont des polymères - et non pas des petites molécules - qui sont insolubles dans de nombreux solvants, notamment les solvants apolaires. Ainsi, l'utilisation d'un procédé alternatif mettant en oeuvre du triacétoxyborohydrure de sodium n'a pas été possible. L'utilisation de borohydrure de sodium dans l'acide sulfurique aqueux et le mélange pyridine-borane s'est également révélée incompatible avec les polymères cationiques. Il apparaissait donc nécessaire de mettre au point un procédé alternatif compatible avec les polymères cationiques de type polyalkylènimines, et qui n'implique que des réactifs pharmaceutiquement acceptables.

Il a ainsi été trouvé qu'il est possible de préparer des polyalkylènimines fonctionnalisés par traitement d'un polyalkylènimine par un hémiacétal fonctionnalisé en présence d'isopropoxyde de titane (IV) et de borohydrure de sodium.

Un tel procédé présente l'avantage de pouvoir être mis en oeuvre dans un solvant compatible avec les polyalkylènimines comme par exemple les alcools et n'implique que des réactifs à la fois moins coûteux et peu toxiques.

Dans différents articles de Bhattacharyya et al. *(J. Org. Chem.,* 1995, 60, pp. 4928-4929 ; *Synlett,* 1995, pp. 1079-1080 ; *J. Chem. Soc.,* Perkin Trans. 1, 1998, pp. 2527-2531) le procédé de préparation d'amines à partir de cétones ou d'aldéhydes suivant avait été décrit : 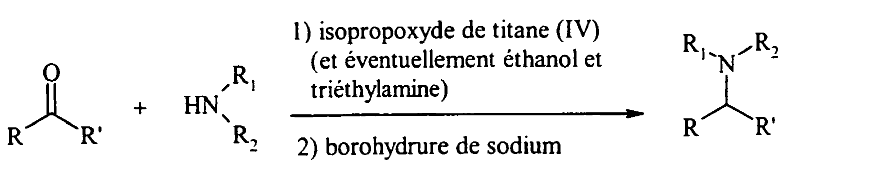 où R et R' représentent indépendamment l'un de l'autre un atome d'hydrogène, un groupe alkyle, un aryle ou forment ensemble un groupe cycloalkyle à 5, 6 ou 7 chaînons contenant éventuellement un hétéroatome, et R₁ et R₂ représentent indépendamment l'un de l'autre un atome d'hydrogène, un groupe alkyle éventuellement substitué par un hydroxy ou un ester, ou bien R₁ et R₂ forment ensemble un groupe cycloalkyle à 5 ou 6 chaînons contenant éventuellement un hétéroatome. Cependant, un tel procédé n'était décrit que dans le cadre de la préparation de petites molécules, c'est-à-dire en particulier des molécules non-polymériques, contenant une seule fonction amine à partir de la cétone ou de l'aldéhyde correspondant.

Selon la présente invention, le polyalkylènimine de départ a pour formule générale : 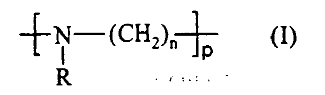 dans laquelle R représente un atome d'hydrogène ou bien un groupe de formule générale : 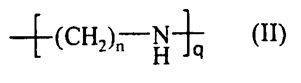 n est un nombre entier compris entre 2 et 10 inclus,
et p et q sont des nombres entiers, étant entendu que la somme p+q est telle que le poids moléculaire moyen du polymère est compris entre 100 et 10⁷ Da inclus.

Il est entendu que dans la formule générale (I), la valeur de n et le groupe R peuvent varier entre les différents motifs -NR-(CH₂)ₙ- et -(CH₂)ₙ-NH-. Ainsi, la formule générale (I) regroupe à la fois des polymères linéaires et des polymères branchés, ainsi que des homopolymères et des hétéropolymères.

De préférence, n est compris entre 2 et 5. Des polymères préférés sont par exemple le polyéthylènimine (PEI) ou le polypropylènimine (PPI). En outre, les polymères préférés pour la mise en oeuvre de la présente invention et qui se sont montrés tout particulièrement efficaces en transfection sont ceux dont le poids moléculaire moyen est compris entre 10³ et 5.10⁶. A titre d'exemple, on peut citer le polyéthylènimine de poids moléculaire moyen 50 000 Da (PEI 50K), 25 000 Da (PEI 25K), 22 000 Da (PEI 22K), ou encore le polypropylènimine 800 000 Da (PPI 800K).

Les polyalkylènimines utilisés dans la présente invention peuvent être obtenus selon différentes méthodes connues de l'homme de l'art. Ils peuvent par exemple être synthétisés chimiquement à partir du ou des monomères correspondants, en condition de polymérisation anionique (par exemple polymérisation de l'éthylènimine), ou par réduction de polyamides obtenus par polycondensation de diacides ou de diamines, ou encore par réduction d'imines obtenues par polycondensation de dialdéhydes avec des diamines. En outre, de nombreux polyalkylènimines sont accessibles commercialement, comme par exemple le PEI 25K, le PEI 22K ou encore le PPI 800K.

On entend au sens de l'invention par « polyalkylènimine fonctionnalisé » des polymères cationiques de type polyalkylèimine sur lesquels des éléments de ciblage sont liés de façon covalente. Ces éléments de ciblage permettent d'orienter le transfert de l'acide nucléique vers certains types cellulaires, certains tissus ou encore certains compartiments cellulaires souhaités. La liaison covalente entre l'élément de ciblage et le polyalkylènimine est obtenue par réaction avec un hémiacétal fonctionnalisé, c'est-à-dire un hémiacétal dont l'un des substituants est ledit élément de ciblage, dans les conditions réactionnelles énoncées précédemment.

Plus précisément, on entend par hémiacétal fonctionnalisé au sens de la présente invention, toute molécule ayant pour formule générale : 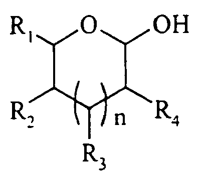 dans laquelle n est égal à 0 ou 1, et R₁, R₂, R₃ et R₄ sont identiques ou différents et représentent indépendamment les uns des autres un atome d'hydrogène, un groupe compatible avec la réaction mise en oeuvre ou bien un élément de ciblage, étant entendu que un et un seul substituant parmi R₁, R₂, R₃ et R₄ est un élément de ciblage.

A titre d'exemple de groupe compatible, on peut citer les hydroxy, les alkyles ayant 1 à 4 atomes de carbone (1 à 4 C) ou encore les hydroxyalkyles (1 à 4 C).

L'élément de ciblage peut être soit directement greffé sur l'hémiacétal, soit il est greffé par l'intermédiaire d'une molécule de liaison bifonctionnelle (également appelée « linker »). Le « linker » permet le greffage dudit élément de ciblage sur l'hémiacétal, greffage qui pourrait autrement ne pas être possible d'un point de vue chimique, et/ou permet d'éloigner ledit élément de ciblage de l'hémiacétal. Par molécule de liaison bifonctionnelle, on entend toute molécules comportant au moins une fonction qui peut se lier covalemment à l'élément de ciblage et au moins une fonction qui peut se lier covalemment à l'hémiacétal.

Au sens de l'invention, on entend par élément de ciblage toute molécule qui permet d'orienter le transfert de l'acide nucléique. Cet élément de ciblage peut être un élément de ciblage extracellulaire permettant d'orienter le transfert de l'ADN vers certains, types cellulaires ou certains tissus souhaités (cellules tumorales, cellules hépatiques, cellules hématopoiétiques...). Il peut également s'agir d'un élément de ciblage intracellulaire permettant d'orienter le transfert de l'acide nucléique vers certains compartiments cellulaires privilégiés (mitochondries ou noyau par exemple).

Parmi les éléments de ciblage utilisables dans le cadre de l'invention, on peut citer les sucres, les peptides, les protéines, les oligonucléotides, les lipides, les neuromédiateurs, les hormones, les vitamines ou leurs dérivés. Préférentiellement, il s'agit par exemple de sucres, de peptides ou de protéines tels que des anticorps ou des fragments d'anticorps, des ligands de récepteurs cellulaires ou des fragments de ceux-ci, des récepteurs ou encore des fragments de récepteurs. En particulier, il peut s'agir de ligands de récepteurs de facteurs de croissance, de récepteurs de cytokines, de récepteurs de type lectines cellulaires, ou de ligands à séquence RGD avec une affinité pour les récepteurs de protéines d'adhésion comme les intégrines. On peut également citer les récepteurs de la transferrine, des HDL et des LDL, ou le transporteur du folate. L'élément de ciblage peut également être un sucre permettant de cibler des lectines tels que les récepteurs aux asialoglycoprotéines ou aux syalylés tel que le Sialyl Lewis X, ou encore un fragment Fab d'anticorps, ou un anticorps simple chaîne (ScFv). A titre d'exemple, ledit sucre peut être choisi parmi les mono-, di- ou tri-saccharides. Il peut s'agir par exemple de galactose, mannose, fucose, rhamnose, lactose, ou encore de maltose.

Généralement, on effectue la réaction dans un solvant alcoolique, par exemple le méthanol ou l'éthanol, à température comprise entre 100°C et 30°C. De préférence, on opère dans l'éthanol à température ambiante, c'est-à-dire à température comprise entre 18°C et 25°C.

En outre, on utilise généralement entre 25 et 100 moles d'isopropoxyde de titane (IV) par mole de polymère introduit, et plus préférentiellement entre 40 et 60 moles d'isopropoxyde de titane (IV) par mole de polymère.

On introduit également dans le mélange réactionnel une quantité de borohydrure de sodium égale à 50 à 80 % (molaire) de la quantité d'isopropoxyde de titane (IV) utilisée.

La quantité d'hémiacétal fonctionnalisé utilisée pour la mise en oeuvre du procédé selon l'invention dépend du taux de greffage que l'on souhaite obtenir. Avantageusement, on utilise entre 6 et 100 moles d'hémiacétal fonctionnalisé par mole de polymère.

Selon la quantité d'hémiacétal fonctionnalisé mis en oeuvre, on obtient des polyalkylènimines fonctionnalisés à des taux qui peuvent varier entre 1% et 20%, et de préférence entre 4% et 20%. Dans le cas de l'utilisation d'un sucre à titre d'élément de ciblage, le pourcentage de sucres greffés sur le polyalkylènimine peut être déterminé précisément en utilisant la méthode au résorcinol. Cette méthode consiste à traiter le polyalkylènimine fonctionnalisé qui a été préalablement dialysé avec du résorcinol et de l'acide sulfurique, puis on chauffe à 90°C pendant 20 minutes. Après refroidissement, l'absorbance à 495 nm est mesurée et comparée à un échantillon de référence (solution standard). La dialyse permet d'éliminer les éléments de ciblage non-greffés, et la mesure faite en comparaison d'un échantillon de référence permet de déterminer la quantité d'éléments de ciblage greffés. Cette méthode constitue par ailleurs l'un des moyens les plus sûrs pour caractériser le produit obtenu, car s'agissant de polymères, il n'est pas possible de les caractériser par RMN (Résonnance Magnétique Nucléaire) ou par mesure de masse comme cela se fait classiquement sur des petites molécules.

Le procédé selon la présente invention est particulièrement intéressant, car il constitue un procédé alternatif à la fois peu coûteux et peu toxique, pour préparer des vecteurs de transfert d'acides nucléiques capables de cibler certains tissus, certains types cellulaires ou certains compartiments cellulaires spécifiques. En outre, il a été remarqué que les polyalkylènimines fonctionnalisés par des sucres sont moins toxiques vis-à-vis des cellules que les polyalkylènimines non-fonctionnalisés.

Les polyalkylènimines fonctionnalisés sont utiles pour la transfection d'acides nucléiques dans les cellules. Dans ce but, les polyalkylènimines fonctionnalisés sont mélangés à un ou plusieurs acides nucléiques de façon à former des complexes également appelés « polyplexes » (on peut alors parler de « polyfection » plutôt que de transfection). Afin d'obtenir une efficacité de transfection optimale, les proportions de polyalkylènimine fonctionnalisé et d'acide nucléique sont choisies de préférence de sorte que le polyplexe formé est globalement neutre ou légèrement positif. D'une manière générale, on choisit lesdites proportions de façon à former des polyplexes positifs et ne précipitant pas, sans pour autant augmenter de façon trop importante la cationicité des polyplexes formés car cela augmente aussi la toxicité. Lesdites proportions doivent ainsi être déterminées au cas par cas. Cependant, lesdites proportions sont généralement choisies de sorte que le rapport molaire entre les amines du polyalkylènimine fonctionnalisé et les phosphates de l'acide nucléique est compris entre 0,1 et 50, de préférence entre 0,5 et 20. Bien entendu, ce rapport peut être aisément adapté et optimisé par l'homme du métier en fonction du polyalkylènimine fonctionnalisé utilisé, de l'acide nucléique, de la cellule cible, du mode d'administration, ou encore selon les applications envisagées (notamment le type de cellules à transfecter).

Dans les polyplexes tels que décrits ci-avant, l'acide nucléique peut être aussi bien un acide désoxyribonucléique qu'un acide ribonucléique. Il peut s'agir de séquences naturelles ou artificielles, et notamment d'ADN génomique (ADNg), d'ADN complémentaire (ADNc), d'ARN messager (ARNm), d'ARN de transfert (ARNt), d'ARN ribosomique (ARNr), de séquences hybrides ou de séquences synthétiques ou semi-synthétiques, d'oligonucléotides modifiés ou non. Ces acides nucléiques peuvent être par exemple d'origine humaine, animale, végétale, bactérienne, ou virale. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement.

Concernant plus particulièrement les acides désoxyribonucléiques, ils peuvent être simple ou double brin de même que des oligonuléotides courts ou des séquences plus longues. En particulier, les acides nucléiques sont avantageusement constitués par exemple par des plasmides, des vecteurs, des épisomes, ou encore des cassettes d'expression. Ces acides désoxyribonucléiques peuvent notamment porter une origine de réplication fonctionnelle ou non dans la cellule cible, un ou plusieurs gènes marqueurs, des séquences régulatrices de la transcription ou de la réplication, des gènes d'intérêt thérapeutique, des séquences antisens modifiées ou non, ou encore des régions de liaison à d'autres composants cellulaires.

De préférence, l'acide nucléique comprend un ou plusieurs gènes d'intérêt thérapeutique sous contrôle de séquences de régulation, par exemple un ou plusieurs promoteurs et un terminateur transcriptionnel actifs dans les cellules cibles.

Au sens de l'invention, on entend par gène d'intérêt thérapeutique notamment tout gène codant pour un produit protéique ayant un effet thérapeutique. Le produit protéique ainsi codé peut être notamment une protéine ou un peptide. Ce produit protéique peut être exogène homologue ou endogène vis-à-vis de la cellule cible, c'est-à-dire un produit qui est normalement exprimé dans la cellule cible lorsque celle-ci ne présente aucune pathologie. Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène d'intérêt thérapeutique peut aussi coder pour un mutant d'une protéine cellulaire, ayant par exemple une stabilité accrue ou une activité modifiée. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut à titre d'exemple citer les enzymes, les dérivés sanguins, les hormones, les lymphokines (par exemple les interleukines, les interférons, ou le TNF : FR 92/03120), les facteurs de croissance, les neurotransmetteurs ou leurs précurseurs ou enzymes de synthèse, les facteurs trophiques (par exemple BDNF, CNTF, NGF, IGF, GMF, aFGF, bFGF, VEGF, NT3, NT5, ou HARP/pléiotrophine) les apolipoprotéines (par exemple ApoAI, ApoAIV, ou ApoE : FR 93/05125), la dystrophine ou une minidystrophine (FR 91/11947), la protéine CFTR associée à la mucoviscidose, les gènes suppresseurs de tumeurs (par exemple p53, Rb, Rap1A, DCC, ou k-rev : FR 93/04745), les gènes codant pour des facteurs impliqués dans la coagulation (par exemple les Facteurs VII, VIII, IX), les gènes intervenant dans la réparation de l'ADN, les gènes suicides (thymidine kinase, cytosine déaminase), les gènes de l'hémoglobine ou d'autres transporteurs protéiques, les enzymes du métabolisme ou du catabolisme.

L'acide nucléique d'intérêt thérapeutique peut également être un gène ou une séquence antisens, dont l'expression dans la cellule cible permet de contrôler l'expression de gènes ou la transcription d'ARNm cellulaires. De telles séquences peuvent par exemple être transcrites dans la cellule cible en ARN complémentaires d'ARNm cellulaires et bloquer ainsi leur traduction en protéine, selon la technique décrite dans le brevet EP 140 308. Les gènes thérapeutiques comprennent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capables de générer chez l'homme ou l'animal une réponse immunitaire. Dans ce mode particulier de mise en oeuvre, l'invention permet la réalisation soit de vaccins soit de traitements immunothérapeutiques appliqués à l'homme ou à l'animal, notamment contre des micro-organismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus", d'autres virus ou encore de peptides antigéniques spécifiques de tumeurs (EP 259 212).

Préférentiellement, l'acide nucléique comprend également des séquences permettant l'expression du gène d'intérêt thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, ou RSV. En outre, ces séquences d'expression peuvent être modifiées par exemple par addition de séquences d'activation ou de régulation. Il peut aussi s'agir de promoteur, inductible ou répressible.

Les polyplexes ainsi formés peuvent être formulés en vue d'administrations par exemple par voie topique, cutanée, orale, rectale, vaginale, parentérale, intranasale, intraveineuse, intramusculaire, sous-cutanée, intraoculaire, transdermique, intratrachéale, ou intrapéritonéale. De préférence, les polyplexes formés contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acides nucléiques utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus, par exemple au niveau des tumeurs, ou dans les voies circulatoires, soit d'un traitement de cellules en culture suivi de leur réimplantation *in vivo,* par injection ou greffe. Les tissus concernés dans le cadre de la présente invention sont par exemple les muscles, la peau, le cerveau, les poumons, le foie, la rate, la moelle osseuse, le thymus, le coeur, la lymphe, le sang, les os, les cartilages, le pancréas, les reins, la vessie, l'estomac, les intestins, les testicules, les ovaires, le rectum, le système nerveux, les yeux, les glandes, ou les tissus conjonctifs.

Les compositions comprenant les polyplexes tels que décrits ci-avant peuvent être utilisées pour le transfert d'acides nucléiques dans les cellules. Plus précisément, lesdites compositions peuvent être utilisées pour la préparation d'un médicament destiné à traiter les maladies, en particulier les maladies qui résultent d'une déficience en un produit protéique ou nucléique.

Une méthode pour transférer des acides nucléiques dans des cellules consiste à effectuer les étapes suivantes :
(1) la formation d'une composition comprenant les polyplexes tels que décrits ci-avant, et
(2) la mise en contact des cellules avec la composition formée en (1).

Outre les dispositions qui précèdent, la présente invention comprend également d'autres caractéristiques et avantages qui ressortiront des exemples et figures qui suivent, et qui doivent être considérés comme illustrant l'invention sans en limiter la portée.

### FIGURES

**Figure 1 :** histogramme représentant l'activité luciférase en RLU/µg de protéines (RLU : « Relative Light Unit » c'est-à-dire unité de lumière relative) dans les cellules ECV304 pour différentes formulations polyalkylènimine glycosylé/ADN à des rapports de charges (+/-) variant de 3 à 28.
   La courbe en trait continu noir représente le pourcentage de survie cellulaire selon le rapport de charges (+/-) entre le polyalkylènimine glycosylé et l'ADN pour chaque formulation [3a], [3b], [4a] et [4b] utilisée.
**Figure 2 :** histogramme représentant l'activité luciférase en RLU/µg de protéines (RLU : « Relative Light Unit » c'est-à-dire unité de lumière relative) dans les cellules HeLa pour différentes formulations polyalkylènimine glycosylé/ADN à des rapports de charges (+/-) variant de 3 à 28.
   La courbe en trait continu noir représente le pourcentage de survie cellulaire selon le rapport de charges (+/-) entre le polyalkylènimine glycosylé et l'ADN pour chaque formulation [3a], [3b], [4a] et [4b] utilisée.
**Figure 3 :** histogramme représentant l'activité luciférase en RLU/µg de protéines (RLU : « Relative Light Unit » c'est-à-dire unité de lumière relative) dans les cellules HepG2 pour différentes formulations polyalkylènimine glycosylé/ADN à des rapports de charges (+/-) variant de 3 à 28.
   La courbe en trait continu noir représente le pourcentage de survie cellulaire selon le rapport de charges (+/-) entre le polyalkylènimine glycosylé et l'ADN pour chaque formulation [3a], [3b], [4a] et [4b] utilisée.
**Figure 4:** Représentation schématique du plasmide pXL2774 utilisé dans les expériences de transfert d'ADN dans les cellules.

### EXEMPLES

### Exemple 1 : préparation de PEI-maltose 4% [3a]

La réaction mise en oeuvre peut être écrite schématiquement de la façon suivante :

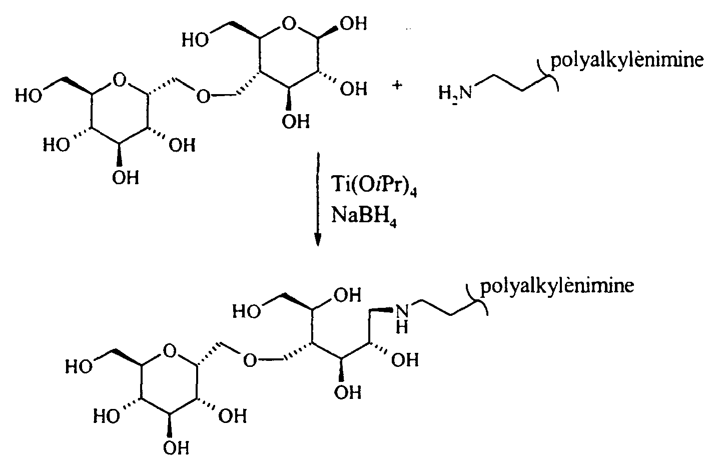

0,02 mmol de PEI 25 KDa (500 mg, de chez Aldrich) sont dissoutes dans 20 ml d'éthanol anhydre. 0,7 mmol de maltose (252 mg) sont ensuite ajoutées et la solution ainsi obtenue est mélangée sous atmosphère d'azote pendant 15 minutes. 1 mmol d'isopropoxyde de Titane IV (0,3 ml) est ajoutée lentement au mélange réactionnel et on maintient sous agitation pendant une nuit. Puis on ajoute 0,75 mmol de borohydrure de sodium (28,5 mg) et on continue l'agitation pendant 8 heures. Le mélange réactionnel est ensuite filtré et concentré à 10 ml. La solution résultante est finalement dialysée pendant 12 heures (membrane d'exclusion de taille 12000). Le rendement est de 80%.

Le pourcentage de résidus de maltose greffés est déterminé par la méthode au résorcinol précédemment décrite : 23 résidus maltose se sont greffés sur le PEI 25 KDa, correspondant à un taux de greffage des groupements amino de 4 %.

### Exemple 2 : préparation de PEI-maltose 12% [3b]

0,02 mmol de PEI 25 KDa (500 mg, de chez Aldrich) sont dissoutes dans 20 ml d'éthanol anhydre. 2 mmol de maltose (720 mg) sont ensuite ajoutées et la solution ainsi obtenue est mélangée sous atmosphère d'azote pendant 15 minutes. 2,7 mmol d'isopropoxyde de Titane IV (0,8 ml) sont ajoutées lentement au mélange réactionnel et on maintient sous agitation pendant une nuit. Puis on ajoute 2 mmol de borohydrure de sodium (76 mg) et on continue l'agitation pendant 8 heures. Le mélange réactionnel est ensuite filtré et concentré à 10 ml. La solution résultante est finalement dialysée pendant 12 heures (membrane d'exclusion de taille 12000). Le rendement est de 80%.

Le pourcentage de résidus de maltose greffés est déterminé par la méthode au résorcinol précédemment décrite : 70 résidus maltose se sont greffés sur le PEI 25 KDa, correspondant à un taux de greffage des groupements amino de 12 %.

### Exemple 3 : préparation de PEI-lactose 6% [4a]

La réaction mise en oeuvre peut être écrite schématiquement de la façon suivante :

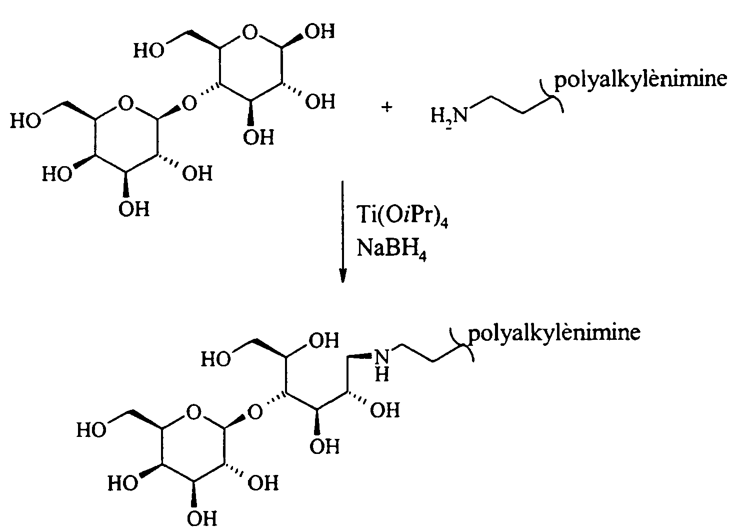

0,02 mmol de PEI 25 KDa (500 mg, de chez Aldrich) sont dissoutes dans 20 ml d'éthanol anhydre. 1 mmol de lactose (360 mg) est ensuite ajoutée et la solution ainsi obtenue est mélangée sous atmosphère d'azote pendant 15 minutes. 1,35 mmol d'isopropoxyde de Titane IV (0,4 ml) sont ajoutées lentement au mélange réactionnel et on maintient sous agitation pendant une nuit. Puis on ajoute 1 mmol de borohydrure de sodium (38 mg) et on continue l'agitation pendant 8 heures. Le mélange réactionnel est ensuite filtré et concentré à 10 ml. La solution résultante est finalement dialysée pendant 12 heures (membrane d'exclusion de taille 12000). Le rendement est de 80%.

Le pourcentage de résidus de lactose greffés est déterminé par la méthode au résorcinol précédemment décrite : 35 résidus lactose se sont greffés sur le PEI 25 KDa, correspondant à un taux de greffage des groupements amino de 6 %.

### Exemple 4 : préparation de PEI-lactose 17% [4b]

0,02 mmol de PEI 25 KDa (500 mg, de chez Aldrich) sont dissoutes dans 20 ml d'éthanol anhydre. 3 mmol de lactose (1080 mg) sont ensuite ajoutées et la solution ainsi obtenue est mélangée sous atmosphère d'azote pendant 15 minutes. 4 mmol d'isopropoxyde de Titane IV (1,2 ml) sont ajoutées lentement au mélange réactionnel et on maintient sous agitation pendant une nuit. Puis on ajoute 3 mmol de borohydrure de sodium (114 mg) et on continue l'agitation pendant 8 heures. Le mélange réactionnel est ensuite filtré et concentré à 10 ml. La solution résultante est finalement dialysée pendant 12 heures (membrane d'exclusion de taille 12000). Le rendement est de 80%.

Le pourcentage de résidus de lactose greffés est déterminé par la méthode au résorcinol précédemment décrite : 99 résidus lactose se sont greffés sur le PEI 25 KDa, correspondant à un taux de greffage des groupements amino de 17 %.

### Exemple d'utilisation : tansfection in vitro d'ADN plasmidique complexé au PEI-maltose 4% et 12% ou au PEI-lactose 6% et 17% dans différents types cellulaires.

Cet exemple illustre la capacité des polyalkylènimines fonctionnalisés synthétisés ci-avant à transfecter l'ADN dans les cellules.

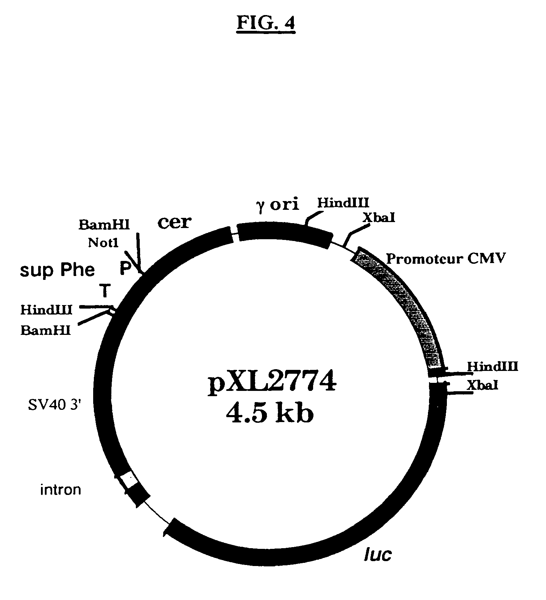

L'ADN utilisé est le plasmide pXL2774 en solution dans un mélange de chlorure de sodium 150 mM à une concentration de 80 µg/ml. Ce plasmide contient le gène luc codant pour la luciférase sous contrôle du promoteur CMV du cytomégalovirus. Sa taille est de 4500 bp. Le schéma de ce plasmide est représenté à la figure 4. Le plasmide pXL2774 a été purifié selon les méthodes décrites dans la demande de brevet WO 97/35002.

Les polyplexes sont prépares par mélange volume à volume d'une solution de plasmide pXL2774 avec une solution de PEI fonctionnalisé dilué dans l'eau à des concentrations variables selon le rapport de charge souhaité.

Des microplaques de 24 puits sont ensemencées avec 60000 cellules HeLa (ATCC) par puit, et transfectées 24 heures plus tard. 50 µl de la solution de polyplexes sont ajoutés goutte à goutte dans chaque puit. En l'absence de sérum, le milieu était au préalable supplémenté par du SVF (sérum de veau foetal) 2 heures avant la transfection. Les cellules sont ensuite incubées à 37°C pendant 4 heures. Le milieu contenant les complexes est ensuite enlevé et remplacé par un mélange de DMEM et de 10% de sérum de veau foetal. Puis, les cellules sont à nouveau mises en culture pendant 24 heures. Enfin, les cellules sont lysées et testées en utilisant un kit de test de luciférase (Promega) et un luminomètre Dynex MLX. En outre, la toxicité des polyplexes a été évaluée par mesure des concentrations du lysat cellulaire et exprimée en activité enzymatique par µg de protéines du lysat.

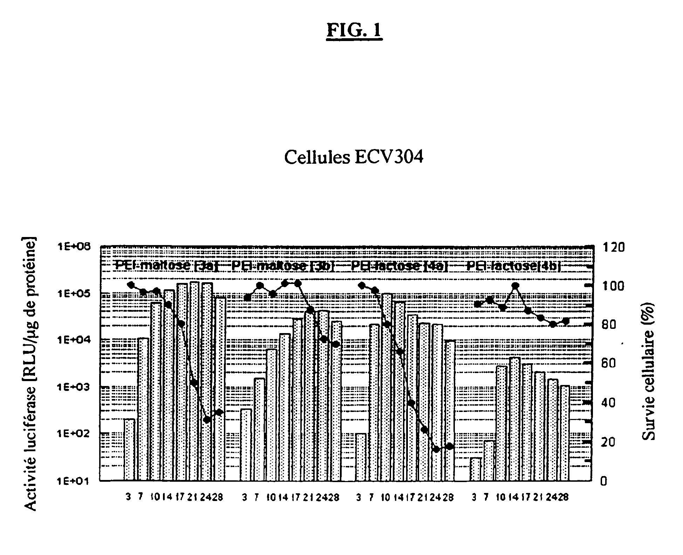

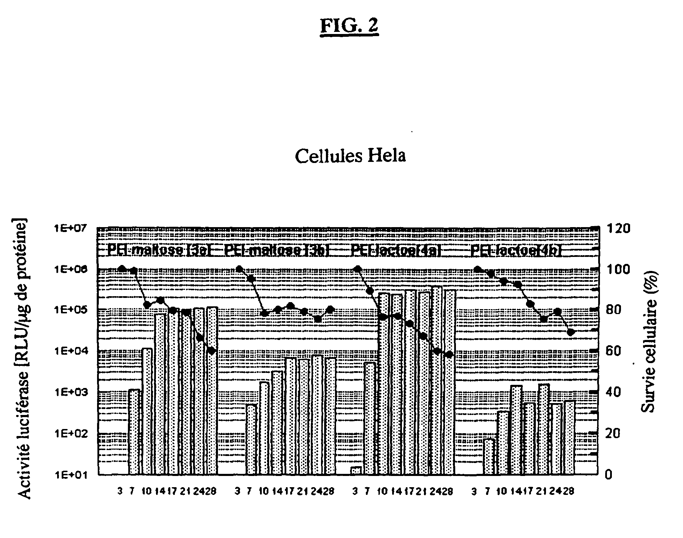

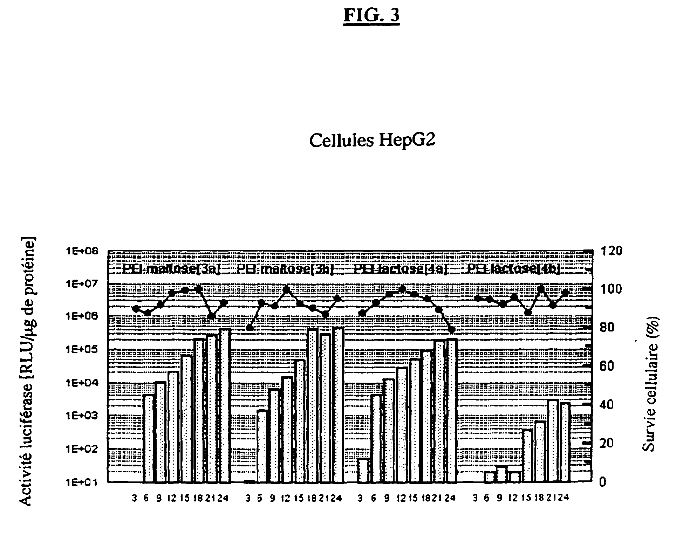

Les résultats indiqués aux figures 1, 2 et 3 rendent compte de l'efficacité de transfection avec les différents PEI fonctionnalisés synthétisés ci-avant pour des rapports de charges avec l'ADN variables et dans 3 lignées cellulaires différentes.

On constate globalement que l'efficacité de transfert est relativement élevée sauf lorsque l'on utilise des polyplexes formés à partir de PEI à haut taux de greffage (efficacité avec le PEI-maltose 12% moins élevée qu'avec le PEI-maltose 4% et efficacité avec le PEI-lactose 17% moins élevée qu'avec le PEI-lactose 6%). Le taux de greffage est donc un paramètre qu'il est important d'optimiser selon les applications envisagées.

En outre, il a été constaté que la substitution des groupes amino par un hémiacétal fonctionnalisé réduit de façon significative la toxicité induite par le PEI vis-à-vis des cellules, en particulier à rapport de charge avec l'ADN élevé. En effet, le pourcentage de survie des cellules (figures 1, 2 et 3) reste beaucoup plus élevé que celui qu'on obtient avec l'utilisation de PEI non-fonctionnalisé pour transfecter l'ADN (résultat non-montré).

Ainsi, cet exemple montrent que les polyalkylènimines fonctionnalisés obtenus par le procédé selon la présente invention, qui est sûr et peu coûteux , sont de bons candidats pour la transfection d'ADN dans les cellules.

## Revendications

1. Procédé de préparation de polyalkylènimines fonctionnalisés **caractérisé en ce que** l'on traite un polyalkylènimine par un hémiacétal fonctionnalisé en présence d'isopropoxyde de titane (IV) et de borohydrure de sodium.

2. Procédé de préparation de polyalkylènimines fonctionnalisés selon la revendication 1 **caractérisé en ce que** l'on opère dans un solvant alcoolique.

3. Procédé de préparation de polyalkylènimines fonctionnalisés selon la revendication 2 **caractérisé en ce que** ledit solvant alcoolique est le méthanol ou l'éthanol.

4. Procédé de préparation de polyalkylènimines fonctionnalisés selon la revendication 1 **caractérisé en ce que** l'on opère en outre à température comprise entre 10 et 30°C.

5. Procédé de préparation de polyalkylènimines fonctionnalisés selon la revendication 1 **caractérisé en ce que** l'on utilise entre 25 et 100 moles d'isopropoxyde de titane (IV) par mole de polyalkylènimine.

6. Procédé de préparation de polyalkylènimines fonctionnalisés selon la revendication 1 **caractérisé en ce que** l'on utilise une quantité de borohydrure de sodium égale à 50 à 80 % (molaire) de la quantité d'isopropoxyde de titane (IV) utilisée.

7. Procédé de préparation de polyalkylènimines fonctionnalisés selon la revendication 1 **caractérisé en ce que** l'on utilise entre 6 et 100 moles d'hémiacétal fonctionnalisé par mole de polyalkylènimine.

8. Procédé de préparation de polyalkylènimines fonctionnalisés selon la revendication 1 **caractérisé en ce que** le polyalkylènimine de départ a pour formule générale : 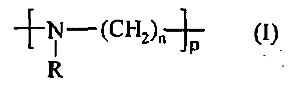 dans laquelle R représente un atome d'hydrogène ou bien un groupe de formule générale : 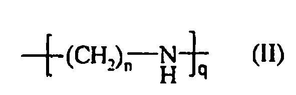 n est un nombre entier compris entre 2 et 10 inclus,
et p et q sont des nombres entiers, étant entendu que la somme p+q est telle que le poids moléculaire moyen du polymère est compris entre 100 et 10⁷ Da inclus.

9. Procédé de préparation de polyalkylènimines fonctionnalisés selon la revendication 8 **caractérisé en ce que** ledit polyalkytènimine de départ est le polyéthylènimine (PEI) ou le polypropylènimine (PPI).

10. Procédé de préparation de polyalkylènimines fonctionnalisés selon la revendication 9 **caractérisé en ce que** ledit polyalkylènimine de départ est choisi parmi le polyéthylènimine de poids moléculaire moyen 50 000 Da ou 25 000 Da ou 22 000 Da ou le polypropylènimine de poids moléculaire moyen 800 000 Da.

11. Procédé de préparation de polyalkylènimines fonctionnalisés selon la revendication 1 **caractérisé en ce que** l'hémiacétal fonctionnalisé a pour formule générale : 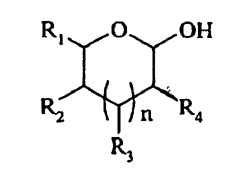 dans laquelle n est égal à 0 ou 1, et R₁, R₂, R₃ et R₄ sont identiques ou différents et représentent indépendamment les uns des autres un atome d'hydrogène, un groupe compatible avec la réaction mise en oeuvre ou bien un élément de ciblage, étant entendu que un et un seul substituant parmi R₁, R₂, R₃ et R₄ est un élément de ciblage.

12. Procédé de préparation de polyalkylènimines fonctionnalisés selon la revendication 11 **caractérisé en ce que** ledit groupe compatible est choisi parmi les hydroxy, les alkyles ayant 1 à 4 atomes de carbone (1 à 4 C) ou encore les hydroxyalkyles (1 à 4 C).

13. Procédé de préparation de polyalkylènimines fonctionnalisés selon la revendication 11 **caractérisé en ce que** ledit élément de ciblage est choisi parmi les sucres, les peptides, les protéines, les oligonucléotides, les lipides, les neuromédiateurs, les hormones, les vitamines ou leurs dérivés.

14. Procédé de préparation de polyalkylènimines fonctionnalisés selon la revendication 13 **caractérisé en ce que** ledit élément de ciblage est choisi parmi les ligands de récepteurs de facteurs de croissance, de récepteurs de cytokines, de récepteurs de type lectines cellulaires, les ligands à séquence RGD avec une affinité pour les récepteurs de protéines d'adhésion comme les intégrines, les récepteurs de la transferrine, les HDL les LDL, le transporteur du folate, le Sialyl Lewis X, les fragments Fab d'anticorps, les anticorps simple chaîne (ScFv), les mono-, di- ou les tri-saccharides.

15. Procédé de préparation de polyalkylènimines fonctionnalisés selon la revendication 14 **caractérisé en ce que** ledit saccharide est choisi parmi le galactose, le mannose, le fucose, le rhamnose, le lactose, ou encore le maltose.

16. Procédé de préparation de polyalkylènimines fonctionnalisés selon la revendication 1 **caractérisé en ce que** le pourcentage d'hémiacétals fonctionnalisés greffés sur le polyalkylènimine est compris entre 1% et 20%.

## Claims

1. A method for preparation of functionalized polyalkyleneimines, **characterized in that** a polyalkyleneimine is treated with a functionalized hemiacetal in the presence of titanium (IV) isopropoxide and sodium borohydride.

2. The method for preparation of functionalized polyalkyleneimines according to claim 1 **characterized in that** one operates in an alcoholic solvent.

3. The method for preparation of functionalized polyalkyleneimines according to claim 2, **characterized in that** the said alcoholic solvent is methanol or ethanol.

4. The method for preparation of functionalized polyalkyleneimines according to claim 1 **characterized in that** additionally one operates at a temperature between between 10 and 30 °C.

5. The method for preparation of functionalized polyalkyleneimines according to claim 1, **characterized in that** between 25 and 100 moles of titanium (IV) isopropyloxide are used per mole of polyalkyleneimine.

6. The method for preparation of functionalized polyalkyleneimines according to claim 1, **characterized in that** equal to 50% and 80% (molar) of sodium borohydride are utilized relative to the quantity of titanium (IV) isopropoxide utilized.

7. The method for preparation of functionalized polyalkyleneimines according to claim 1, **characterized in that** between 6 and 100 moles of functionalized hemiacetal are utilized per mole of polyalkyleneimine.

8. The method for preparation of functionalized polyalkyleneimines according to claim 1, **characterized in that** the starting polyalkyleneimine has the general formula: 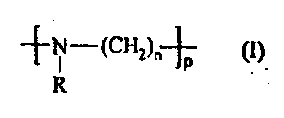 in which R represents a hydrogen atom or else a group of general formula: 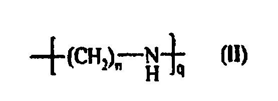 n is a whole number between 2 and 10,
and p and q are whole numbers, it being understood that the sum p + q is such that the average molecular weight of the polymer is between 100 and 10⁷ Da.

9. The method for preparation of functionalized polyalkyleneimines according to claim 8, **characterized in that** the said starting polyalkyleneimine is the polyethyleneimine (PEI) or the polypropyleneimine (PPI).

10. The method for preparation of functionalized polyalkyleneimines according to claim 9, **characterized in that** the starting polyalkyleneimine is chosen among the polyethyleneimine of average molecular weight 50,000 or 25,000 Da or 22,000 Da or the polypropyleneimine of average molecular weight 800,000 Da.

11. The method for preparation of functionalized polyalkyleneimines according to claim 1, **characterized in that** the functionalized hemiacetal has as its general formula: 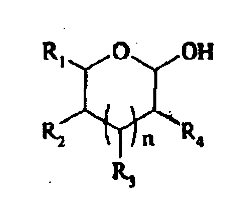 in which n is equal to 0 or 1, and R₁, R₂, R₃ and R₄ are identical or different and independently represent one or the other a hydrogen atom, a group compatible with the reaction taking place or else a target element, it being understood that one single substituant among R₁, R₂, R₃ and R₄ is a target element.

12. The method for preparation of functionalized polyalkyleneimines according to claim 11, **characterized in that** the said compatible group is chosen among the hydroxyl, the alkyls having 1 to 4 carbon atoms (1 to 4 C) or also the hydroxyalkyls (1 to 4 C).

13. The method for preparation of functionalized polyalkyleneimines according to claim 11, **characterized in that** the said target element is chosen among the sugars, the peptides, the proteins, the oligonucleotides, the lipids, the neuro-mediators, the hormones, the vitamins or their derivatives.

14. The method for preparation of functionalized polyalkyleneimines according to claim 13, **characterized in that** the said target element is chosen among the ligands of receptors of growth factors, of cytokine receptors, of cellular lectine type receptors, RGD sequence ligands with an affinity for the receptors of adhesion proteins like the integrines, the transferrine receptors, the HDL, the LDL, the folate transporter, the Sialyl Lewis X, antibody Fab fragments, the simple chain antibodies (ScFv), the mono, di or tri-saccharides.

15. The method for preparation of functionalized polyalkyleneimines according to claim 14, **characterized in that** the said saccharide is chosen among galactose, mannose, fucose, rhamnose, lactose or also maltose.

16. The method for preparation of functionalized polyalkyleneimines according to claim 1, **characterized in that** the percentage of functionalized hemiacetals grafted onto the polyalkyleneimine is between 1% and 20%.

## Patentansprüche

1. Verfahren zur Herstellung von funktionalisierten Polyalkyleniminen, **dadurch gekennzeichnet, dass** ein Polyalkylenimin mit einem funktionalisierten Hemiacetal in Gegenwart von Titan(IV)isöpropoxid und Natriumborhydrid behandelt wird.

2. Verfahren zur Herstellung von funktionalisierten Polyalkyleniminen nach Anspruch 1, **dadurch gekennzeichnet, dass** dieses in einem alkoholischen Lösemittel durchgeführt wird.

3. Verfahren zur Herstellung von funktionalisierten Polyalkyleniminen nach Anspruch 2, **dadurch gekennzeichnet, dass** es sich bei dem alkoholischen Lösemittel um Methanol oder Ethanol handelt.

4. Verfahren zur Herstellung von funktionalisierten Polyalkyleniminen nach Anspruch 1, **dadurch gekennzeichnet, dass** dieses ferner bei einer Temperatur zwischen 10 und 30 °C durchgeführt wird.

5. Verfahren zur Herstellung von funktionalisierten Polyalkyleniminen nach Anspruch 1, **dadurch gekennzeichnet, dass** zwischen 15 und 100 Mol Titan(IV)isopropoxid pro Mol Polyalkylenimin verwendet werden.

6. Verfahren zur Herstellung von funktionalisierten Polyalkyleniminen nach Anspruch 1, **dadurch gekennzeichnet, dass** eine Menge an Natriumborhydrid verwendet wird, welche 50 bis 80 % (molar) der verwendeten Menge an Titan(IV)isopropoxid entspricht.

7. Verfahren zur Herstellung von funktionalisierten Polyalkyleniminen nach Anspruch 1, **dadurch gekennzeichnet, dass** zwischen 6 und 100 Mol funktionalisiertes Hemiacetal pro Mol Polyalkylenimin verwendet werden.

8. Verfahren zur Herstellung von funktionalisierten Polyalkyleniminen nach Anspruch 1, **dadurch gekennzeichnet, dass** das anfängliche Polyalkylenimin die allgemeinen Formel 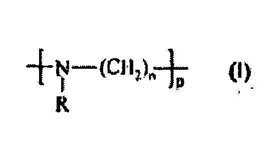 aufweist, wobei R ein Wasserstoffatom darstellt oder auch eine Gruppe der allgemeinen Formel: 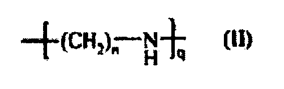 n eine ganze Zahl zwischen 2 und einschließlich 10 ist
und p und q ganzen Zahlen entsprechen, wobei die Summe p+q so gewählt ist, dass das mittlere Molekulargewicht des Polymers zwischen 100 und einschließlich 10⁷ Da liegt.

9. Verfahren zur Herstellung von funktionalisierten Polyalkyleniminen nach Anspruch 8, **dadurch gekennzeichnet, dass** es sich bei dem anfänglichen Polyalkylenimin um Polyethylenimin (PEI) oder Polypropylenimin (PPI) handelt.

10. Verfahren zur Herstellung von funktionalisierten Polyalkyleniminen nach Anspruch 9, **dadurch gekennzeichnet, dass** das anfängliche Polyalkylenimin ausgewählt ist aus Polyethylenimin eines mittleren Molekulargewichts von 50000 Da oder 25000 Da oder 22000 Da oder aus Polypropylenimin eines mittleren Molekulargewichts von 800000 Da.

11. Verfahren zur Herstellung von funktionalisierten Polyalkyleniminen nach Anspruch 1, **dadurch gekennzeichnet, dass** das funktionalisierte Hemiacetal die allgemeine Formel 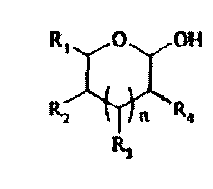 aufweist, wobei n gleich 0 oder 1 ist und R₁, R₂, R₃ und R₄ identisch oder voneinander verschieden sind und unabhängig voneinander ein Wasserstoffatom, eine mit der umgesetzten Reaktion verträgliche Gruppe oder auch ein Zielelement darstellen können, wobei einzig und allein ein Substituent unter R₁, R₂, R₃ und R₄ ein Zielelement ist.

12. Verfahren zur Herstellung von funktionalisierten Polyalkyleniminen nach Anspruch 11, **dadurch gekennzeichnet, dass** die verträgliche Gruppe ausgewählt ist aus Hydroxygruppen, Alkylgruppen, die 1 bis 4 Kohlenstoffatome (1 bis 4C) aufweisen oder auch Hydroxyalkylgruppen (1 bis 4 C).

13. Verfahren zur Herstellung von funktionalisierten Polyalkyleniminen nach Anspruch 11, **dadurch gekennzeichnet, dass** das Zielelement ausgewählt ist aus Zuckern, Peptiden, Proteinen, Oligonukleotiden, Lipiden, Neurotransmittern, Hormonen, Vitaminen oder deren Derivaten.

14. Verfahren zur Herstellung von funktionalisierten Polyalkyleniminen nach Anspruch 13, **dadurch gekennzeichnet, dass** das Zielelement ausgewählt ist aus Liganden von Wachstumsfaktoren, von Cytokinrezeptoren, von Rezeptoren des zellulären Lectintyps, Liganden mit der Sequenz RGD mit einer Affinität für Rezeptoren von Adhäsionsproteinen, wie den Integrinen, Transferrinrezeptoren, HDL und LDL, Folattransporter, Sialyl Lewis X, Fab Fragmenten von Antikörpern, einfachkettigen Antikörpern (ScFv), Mono-, Di- oder Trisacchariden.

15. Verfahren zur Herstellung von funktionalisierten Polyalkyleniminen nach Anspruch 14, **dadurch gekennzeichnet, dass** das Saccharid ausgewählt ist aus Galaktose, Mannose, Fukose, Rhamnose, Laktose oder auch Maltose.

16. Verfahren zur Herstellung von funktionalisierten Polyalkyleniminen nach Anspruch 1, **dadurch gekennzeichnet, dass** der Prozentsatz von auf das Polyalkylenimin aufgepropften funktionalisierten Hemiacetalen zwischen 1 % und 20 % liegt.
